# EUROPEAN PATENT APPLICATION

(11) **EP 4 428 228 A1**
(43) Date of publication of application: **11.09.2024**
(21) Application number: 23305331.3
(22) Date of filing: 10.03.2023
(51) Int. Cl.: C12N 5/076

(54) **NOVEL COMPOSITIONS AND THEIR USE TO INCREASE MOTILITY, VIABILITY AND LIFESPAN OF SPERM CELLS AND FERTILITY**

(71) Applicant: IMV Technologies, 61300 Saint-Ouen-sur-Iton (FR); Ecole Supérieure de Physique et de Chimie Industrielles de la Ville de Paris, 75005 Paris (FR); Centre national de la recherche scientifique, 75016 Paris (FR); SORBONNE UNIVERSITE, 75006 Paris (FR)
(72) Inventor: SCHMITT, Eric, 61300 SAINT-OUEN-SUR-ITON (FR); CARION, Olivier, 61300 SAINT-OUEN-SUR-ITON (FR); PIPART, Perrine, 61300 SAINT-OUEN-SUR-ITON (FR); CAMUGLI, Sabine, 61300 SAINT-OUEN-SUR-ITON (FR); HOURDET, Dominique, 75006 PARIS (FR); TRAN, Yvette, 75005 PARIS (FR)
(74) Representative: Santarelli

(57) **Abstract**

The present invention relates to novel compositions, process for manufacturing said compositions and use thereof for increasing motility, viability and lifespan of sperm cells, and fertility.

## Description

### FIELD OF THE INVENTION

The present invention relates to novel compositions, process for manufacturing said compositions and use thereof for increasing motility, viability and lifespan of sperm cells, and fertility.

### BACKGROUND AND PRIOR ART

Infertility affects one in six couples, with, in one out of two cases, a deficiency of the male partner.

Depending on the severity of the male infertility, various methods to aid couples such as artificial insemination (Al) and in particular intrauterine insemination (IUI) for mild sperm dysfunction, in vitro fertilization (IVF) for moderate sperm dysfunction, and intra-cytoplasmic sperm injection (ICSI) for men with severe sperm dysfunction, may be used.

Freezing of sperm samples is often necessary in such assisted reproduction technology (ART).

However, a major disadvantage of freezing sperm samples is that spermatozoa that have been previously frozen, once thawed, have a reduced motility, viability and lifespan compared to fresh sperm samples, with lower amount of progressive sperm.

Another drawback of the freezing process is an acceleration of the maturation process of sperm, known as capacitation. Capacitation is a necessary step of maturation, normally occurring in the female tract. Although necessary, capacitation leads to irreversible sperm changes and eventually to damages decreasing semen quality, motility and ability to fertilize an oocyte. Capacitation is associated with a strong increase of the metabolic activity and decreases the overall lifespan of sperm within the female reproductive tract. This is particularly damaging when insemination is not synchronized with ovulation.

Moreover, in farm animals, for which artificial insemination (Al) is heavily used, the decreased motility of frozen sperm means that sperm concentration in straws must be increased and this reduces the overall success of the method.

To prevent the drawback linked to the freezing process, it has been found that the use of non-toxic agents can increase sperm motility, viability and lifespan and thus the conception rate in ART methods thereby presenting a strong therapeutic value in the context of medically assisted reproduction not only for therapeutic use in humans, but also for industrial reproduction use in numerous species and in particular animal species.

In particular, it has been surprisingly found that inhibitors of Slo3 potassium channel could be used to increase sperm motility, viability and lifespan and thus the conception rate in ART methods.

However, such agents may be not sufficiently in contact with the sperm thereby impacting their activity and sperm samples may also be subjected to physical degradations such as sedimentation of sperm cells.

There is thus a need to provide novel and non-toxic sperm compositions to be used in ART methods allowing to reinforce the action of agents such as inhibitors of Slo3 potassium channel (and thus also to reduce the amount of agents such as inhibitors of Slo3 potassium channel) when used, to gradually release spermatozoa over time and in particular to ensure a spread of sperm cells in the uterus over a longer period of time during artificial insemination but also to avoid physical degradations such as sedimentation of sperm cells.

In a surprising and unexpected way, the present inventors have found novel and non-toxic compositions which increase the contact time between the agent such as inhibitor of Slo3 potassium channel (without negatively impacting its activity) when used and sperm cells, gradually release spermatozoa over time and in particular ensure a spread of sperm cells in the uterus over a longer period of time during artificial insemination (thereby increasing the insemination window by keeping sperm fertile longer), and also enable avoiding physical degradation such as sedimentation of sperm cells.

### SUMMARY OF THE INVENTION

The present invention relates to a composition comprising:
- human and/or animal semen comprising sperm cells such as spermatozoa,
- a compound selected from collagen peptides, polymers and co-polymers including polysaccharides, and mixtures thereof,
- a semen extender,
- optionally an inhibitor of Slo3 potassium channel, and
- optionally lipids such as liposomes.

The present invention also relates to a gel comprising the composition as previously defined.

The present invention also relates to a kit comprising the composition as previously defined.

The present invention also relates to a process for manufacturing the composition as previously defined or the gel as previously defined comprising the steps of:
- mixing the semen extender with the compound, optionally a metal ion, an inhibitor of Slo3 potassium channel and/or lipids such as liposomes, and optionally the additional compound and/or the divalent or trivalent ion chelating agent, to obtain a mixture,
- diluting said mixture in water to obtain a diluted mixture, and
- mixing the human and/or animal semen to said diluted mixture.

The present invention also relates to the use of the composition as previously defined or of the gel as previously defined to increase the contact time between the sperm cells and the inhibitor of Slo3 potassium channel.

The present invention also relates to the use of the composition as previously defined or to the gel as previously defined to prevent sperm cells from sedimentation.

The present invention also relates to the composition as previously defined or to the gel as previously defined for its use to increase motility, viability and lifespan of sperm cells and in particular spermatozoa, preferably capacitated spermatozoa.

The present invention also relates to the composition as previously defined or to the gel as previously defined for its use to increase fertility.

### DETAILED DESCRIPTION

For the purpose of this application, unless otherwise specified, the ranges of values indicated are inclusive.

The present invention relates to a composition comprising:
- human and/or animal semen comprising sperm cells such as spermatozoa,
- a compound selected from collagen peptides, polymers and co-polymers including polysaccharides, and mixtures thereof,
- a semen extender,
- optionally an inhibitor of Slo3 potassium channel, and
- optionally lipids such as liposomes.

The present inventors have surprisingly found that the composition as previously defined is not toxic and in particular has an anti-crystallization effect during freezing (by inhibiting devitrification of sperm during freezing), enables to prevent sperm cell from oxidation, preserve the integrity of the acrosome of sperm cells, to improve the mitochondrial activity of sperm cells but also the sperm cells motility, viability and lifespan and thus the fertility.

The sperm cells can be fresh sperm cells and in particular freshly ejaculated sperm cells, frozen sperm cells, thawed sperm cells and/or sperm cells recovered from the epididymis, or from the testicle.

By "sperm cells" it is preferably understood spermatozoa.

By "semen extender" it is preferably understood any liquid diluent, preferably aqueous solution, used to increase the volume of the semen until the required dose while preserving the functional characteristics of the sperm cells and in particular their fertilizing ability.

Examples of semen extender include, but are not limited to, mediums comprising one or more compounds selected from sugars such as glucose, galactose, fructose, ribose, trehalose and mixtures thereof, buffering agents such as citrate and in particular sodium citrate, phosphate, bicarbonate and in particular sodium bicarbonate, TES (N-[tris(hydroxymethyl)methyl]-2-aminoethanesulfonic acid), HEPES ((4-(2-hydroxyethyl)-1-piperazineethanesulfonic acid)), MOPS (3-(N-morpholino)propanesulfonic acid), Tris (tris(hydroxymethyl)aminomethane) and mixtures thereof, salts of inorganic ions such as sodium chloride, potassium chloride, sodium tartrate, potassium tartrate, sodium acetate, potassium acetate, sodium sulphate, potassium sulphate and mixtures thereof, protein based compounds such as peptones, milk, lactose, egg yolk, egg phosphatidylcholine (EPC), Bovine Serum Albumin (BSA) and mixtures thereof, chelating agent such as EDTA (Ethylenediaminetetraacetic acid), amino acid such as cysteine, amino acid-based compound such as acetylcysteine, antibiotics such as penicillin and streptomycin, polyvinyl alcohol (PVA), glycerol, detergent, citric acid, and mixtures thereof.

In particular, examples of semen extender may be described for example in Review: Semen extenders used in the artificial insemination of swine, Gadea et al., SPANISH JOURNAL OF AGRICULTURAL RESEARCH 1(2):17-27 and/or Liposomes for cryopreservation of bovine sperm, T Röpke et al., Theriogenology, 2011, Nov;76(8):1465-72*.*

Preferably, examples of semen extender include, but are not limited to, Beltsville Liquid (BL-1), Beltsville Thawing Solution (BTS), Illinois Variable Temperature (IVT), Kiev, Vital^{®}, Acromax^{®}, Androhep^{®}, Androhep^{®} plus, Modena, MR-A^{®}, MULBERRY III^{®}, Reading, X-Cell^{®}, Zorlesco, ZORPVA, SpermAid, Safe Cell Plus, BF-5, BW25, TCF medium, NutriXcell ultra, NutriXcell plus, OptiXcell, OptiXcell Alpha, and mixtures thereof. More preferably, the semen extender is selected from OptiXcell, OptiXcell Alpha, and mixtures thereof.

The polymers and co-polymers including polysaccharides may be anionic polymers and co-polymers including anionic polysaccharides, cationic polymers and co-polymers including cationic polysaccharides, non-ionic polymers and co-polymers including non-ionic polysaccharides, polymers and co-polymers covalently grafted such as polymers and co-polymers grafted with an amine function, thermoresponsive (or temperature-responsive) polymers and co-polymers (such as LCST polymers and copolymers and UCST polymers and copolymers) including polymers and co-polymers grafted from polysaccharides, grafted on polysaccharides or grafted through polysaccharides, and mixtures thereof.

Examples of anionic polymers and co-polymers include, but are not limited to, anionic polysaccharides such as anionic gums and in particular xanthan gum, acacia gum, carrageenan gum, arabinogalactan gum, gellan gum, and mixtures thereof, alginate, pectin, hyaluronic acid (HA), cellulose derivatives such as carboxymethylcellulose (CMC), proteins such as gelatin and ovalbumin, and mixtures thereof

Examples of cationic polymers and co-polymers include, but are not limited to, cationic polysaccharides such as chitosan, cationic derivatives of polysaccharides or cationic modified polysaccharides such as cellulose, dextran and guar gum, proteins such as gelatin and ovalbumin, and mixtures thereof.

Examples of non-ionic polymers and co-polymers include, but are not limited to, non-ionic polysaccharides and in particular microcrystalline cellulose (MCC), cellulose derivatives such as methylcellulose, hydroxymethylcellulose, ethylcellulose, hydroxyethylcellulose, hydroxypropylmethylcellulose (HPMC), and mixtures thereof, scleroglucan, pullulan, ethylene glycol methacrylate polymer (PEGMA), triethylene glycol methacrylate polymer (PTEGMA), oligoethylene glycol methacrylate polymers (POEGMA), Poly(vinylMethylEther) (PVME), Poly(N-isopropylacrylamide) (PNiPAM) and its copolymers, Poly(N-vinylcaprolactam) (PNVCL), poloxamers such as Pluronic^{®} F-127 (CAS 9003-11-6), poly(ethylene oxide-co-propylene oxide) (PEPO), polyoxazoline, and mixtures thereof.

Examples of polymers and copolymers covalently grafted such as polymers and copolymers grafted with an amine function include, but are not limited to, homopolymers and copolymers of N-Substituted poly(acrylamide)s, poly(methacrylamide)s, acrylate polymers and copolymers (such as methacrylate polymers and copolymers), acrylamide polymers and copolymers (such as methacrylamide polymers and copolymers), LCST polymers and copolymers and UCST polymers and copolymers, amino-terminated poloxamers, amino-terminated Poly(N-isopropylacrylamide) (PNiPAM) such as amino-terminated Poly(N-isopropylacrylamide) (PNiPAM) using cysteamine, amino terminated poly(ethylene oxide-co-propylene oxide) (PEPO), amino-terminated Poly(N-vinylcaprolactam) (PNVCL), amino-terminated ethylene glycol methacrylate polymer (PEGMA), amino terminated poly-oligo(ethylene glycol) methyl ether methacrylate polymer (POEGMA), amino-terminated polyoxazolines, and mixtures thereof.

Examples of thermoresponsive polymers (or temperature-responsive polymers) (such as LCST polymers and copolymers and UCST polymers and copolymers) include, but are not limited to, LCST polymers and copolymers, UCST polymers and copolymers, a polymer and/or a co-polymer (side chain) as previously defined grafted to a polysaccharide (backbone) as defined in the present invention, preferably polymers and co-polymers grafted with hyaluronic acid (HA) and/or alginate and in particular sodium alginate such as HA-g-PEPO, Alg-g-PEPO, HA-g-PNiPAm, Alg-g-PNiPAm, HA-g-PEGMA, Alg-g-PEGMA, HA-g-PTEGMA, Alg-g-PTEGMA, HA- PEPO, Alg-PEPO, HA-PNiPAm, Alg-PNiPAm, HA-PEGMA, Alg-PEGMA, HA-PTEGMA, Alg-PTEGMA, and mixtures thereof, more preferably HA-g-PNiPAm, and mixtures thereof.

Examples of LCST polymers include, but are not limited to, N-Substituted poly(acrylamide)s and poly(methacrylamide)s, poly(N-vinyl amide)s, protein related polymers, poly(methyl 2-alkylamidoacrylate)s, poly(oxazoline)s, poly(oxide)s, poly(vinylether)s, poly[oligoethyleneglycol(meth)acrylate]s, poly(phosphoester)s, alkyl-cellulose (such as methycellulose and hydroxypropylmethylcellulose), and mixtures thereof.

Examples of UCST polymers include, but are not limited to, poly(sulfobetaine), N-Substituted poly(acrylamide)s and poly(methacrylamide)s such as poly(N-acryloylglycinamide), poly(N-cyanomethylacrylamide), poly(acrylamide)s and copolymers such as poly(acrylamide-co-acrylonitrile), poly(N-acryloyl asparaginamide), and mixtures thereof.

Preferably, the polymer has a backbone molar mass comprised between 10 000 g/mol and 2 000 000 g/mol, preferably between 50 000 g/mol and 1 000 000 g/mol, preferably between 50 000 g/mol and 400 000 g/mol, more preferably between 100 000 g/mol and 300 000 g/mol and even more preferably between 150 000 and 250 000 g/mol.

Preferably, the polymeric side-chain has a chain molar mass comprised between 500 g/mol and 70 000 g/mol, preferably between 2000 g/mol and 50 000 g/mol and more preferably 2000 g/mol and 40 000 g/mol.

Preferably, the ratio polymeric side-chain and polymer backbone is comprised between 0:100 and 100:0, preferably between 20:80 and 80:20 and more preferably 40:60 and 80:20.

Preferably, the polymer or copolymer including polysaccharides is present at a concentration comprised between 0.25 wt% and 4 wt%, preferably between 0.4 wt% and 3 wt% and more preferably between 0.8 wt% and 2.5 wt% with respect to the total weight of the composition.

Preferably, the polymers and co-polymers as previously defined are biocompatible polymers and co-polymers.

By "biocompatible polymer or co-polymer" it is preferably understood, a polymer and/or a copolymer suitable to be exposed to body and body fluids and able to improve body functions without altering its normal functioning and without triggering allergies or other side effects.

Preferably, the polymers and co-polymers are selected from polysaccharides, gelatin, ethylene glycol methacrylate polymer (PEGMA), triethylene glycol methacrylate polymer (PTEGMA), poly-oligo(ethylene glycol) methyl ether methacrylate polymer (POEGMA), N-isopropylacrylamide polymer (PNiPAM), Poly-N-vinylcaprolactam polymer (PNVCL), poloxamers such as Pluronic^{®} F-127 (CAS 9003-11-6), polyethylene oxide-co-propylene oxide) polymer (PEPO), polymers and co-polymers covalently grafted as previously defined such as polymers and co-polymers grafted with an amine function as previously defined including amino-terminated poloxamers, amino-terminated Poly(N-isopropylacrylamide) (PNiPAM) such as amino-terminated Poly(N-isopropylacrylamide) (PNiPAM) using cysteamine, amino terminated polyethylene oxide-co-propylene oxide) (PEPO), amino-terminated Poly(N-vinylcaprolactam) (PNVCL), amino-terminated ethylene glycol methacrylate polymer (PEGMA), amino terminated poly-oligo(ethylene glycol) methyl ether methacrylate polymer (POEGMA), amino-terminated polyoxazolines, and mixtures thereof such as those compounds grafted from a polysaccharide, grafted on a polysaccharide or grafted through a polysaccharide as previously defined.

Examples of polysaccharides according to the present invention include, but are not limited to, gum such as guar gum, xanthan gum, acacia gum, carrageenan gum, arabinogalactan gum, gellan gum, and mixtures thereof, cellulose such as microcrystalline cellulose (MCC) and cellulose derivatives such as methylcellulose, carboxymethylcellulose (CMC), hydroxymethylcellulose, ethylcellulose, hydroxyethylcellulose, hydroxypropylmethylcellulose (HPMC), and mixtures thereof, alginate such as sodium alginate, pectin, pullulan, chitosan, hyaluronic acid (HA), dextran, scleroglucan, and mixtures thereof.

Pectin may be selected from low-molecular (LM) pectin preferably from citrus pectin, apple pectin, and mixtures thereof.

Examples of mixtures of polysaccharides include, but are not limited to, a mixture of pectin and carboxymethylcellulose (CMC) or a mixture of microcrystalline cellulose (MCC) and carboxymethylcellulose (CMC), and mixtures thereof.

Examples of collagen peptides comprise, but are not limited to, peptan.

Preferably, the collagen peptide is present at a concentration comprised between 0.1 wt% and 10 wt%, preferably between 0.2 wt% and 5 wt% and more preferably between 0.5 wt% and 2.5 wt% with respect to the total weight of the composition.

The composition may further comprise a metal ion, preferably a divalent and/or trivalent metal ion, more preferably selected from beryllium ion, magnesium ion, calcium ion, strontium ion, barium ion, radium ion, and mixtures thereof.

The metal ion may be under the form of an inorganic salt such as chloride and in particular beryllium chloride (BeCl₂), magnesium chloride (MgCl₂), calcium chloride (CaCl₂), strontium chloride (SrCl₂), barium chloride (BaCl₂), radium chloride (RaCl₂), and mixtures thereof.

The metal ion as previously defined may be present in the composition at a concentration ranging from 0.2 mM to 1 M, preferably from 0.5 mM to 80 mM and more preferably from 1 mM and 50 mM.

The compound may be present at a concentration comprised between 0.001 % and 10 % by weight, preferably between 0.01 % and 10 % by weight, and more preferably between 0.1 % and 5 % by weight with respect to the total weight of the composition.

The composition may further comprise one or more additional compounds selected from:
- a sialic acid, and/or
- a saccharide such as mannose, glucosamine and derivatives thereof, and mixtures thereof, and/or
- an oligosaccharide such as oligo(mannose), and/or
- a binding agent having affinity for a sialic acid, and/or
- a binding agent having affinity for a saccharide such as mannose, glucosamine and derivatives thereof, and mixtures thereof, and/or
- a binding agent having affinity for an oligosaccharide such as oligo(mannose), and/or
- an agent, preferably an enzyme, hydrolysing sialic acid, and/or
- an agent, preferably an enzyme, hydrolysing a saccharide such as mannose, glucosamine and derivatives thereof, and mixtures thereof, and/or
- an agent, preferably an enzyme, hydrolysing an oligosaccharide such as oligo(mannose).

Examples of derivatives of glucosamine comprise, but are not limited to, N-acetyl glucosamine (Glc-NAc), β-D(1,3)-galactosamine, and mixtures thereof.

Examples of binding agent having affinity for a sialic acid comprise, but are not limited to, compounds described in the patent application WO 2017012993 (A1), and in particular, monoclonal antibodies, lectins such as Sambucus Nigra Agglutinin (SNA), Triticum Vulgaris Lectin (WGA), Galanthus Nivalis Lectin or Agglutinin (GNL/GNA) with sialic acid interaction, ovalbumin, neo-glycoproteins, and mixtures thereof.

Examples of binding agent having affinity for a saccharide such as mannose, glucosamine and derivatives thereof, and mixtures thereof, and/or having affinity for an oligosaccharide such as oligo(mannose) comprise, but are not limited to, lectin, an antibody, boronic acid and mixtures thereof.

Examples of enzyme hydrolysing sialic acid comprise, but are not limited to, sialidase.

Examples of enzyme hydrolysing a saccharide such as mannose, glucosamine and derivatives thereof comprise, but are not limited to, neuraminidase.

Examples of enzyme hydrolysing an oligosaccharide such as oligo(mannose) comprise, but are not limited to, compounds described in the patent application WO 2017012993 (A1), and in particular transglucosidase, hydrolase compounds, and mixtures thereof.

Preferably, these additional compounds and in particular the lectin, the sialidase and the antibody are natural molecules, preferably molecules that have not been chemically modified, more preferably that have not been modified by a polyethylene glycol (PEG) moiety.

Examples of lectin include, but are not limited to, Wheat Germ Agglutinin (WGA).

The additional compound may be present at a concentration ranging from 0.001 mg/L to 1000 mg/L, preferably from 0.01 mg/L to 100 mg/L and more preferably from 0.02 mg/L to 50 mg/L.

For example, when the additional compound is lectin, it may be present at a concentration ranging from 0.001 mg/L to 100 mg/L, preferably from 0.01 mg/L to 50 mg/L, and more preferably from 0.02 mg/L to 10 mg/L.

For example, when the additional compound is sialic acid, it may be present at a concentration ranging from 0.1 mg/L to 1000 mg/L, preferably from 1 mg/L to 100 mg/L, and more preferably from 10 mg/L to 50 mg/L.

Preferably, the binding agent is a mixture of sialic acid and lectin such as Wheat Germ Agglutinin (WGA).

Preferably, the composition comprises:
- from 0.001 mg/L to 100 mg/L, preferably from 0.01 mg/L to 50 mg/L, and more preferably from 0.02 mg/L to 10 mg/L of lectin, and
- from 0.1 mg/L to 1000 mg/L, preferably from 1 mg/L to 100 mg/L, and more preferably from 10 mg/L to 50 mg/L of sialic acid.

Preferably, the composition comprises:
- human and/or animal semen comprising sperm cells such as spermatozoa,
- a compound selected from collagen peptides, polymers and co-polymers including polysaccharides, and mixtures thereof as previously defined, and preferably scleroglucan, microcrystalline cellulose (MCC), carboxymethylcellulose (CMC), a mixture of microcrystalline cellulose (MCC) and carboxymethylcellulose (CMC), HA-g-PNIPAm, Alg-g-PNIPAm, pectin in combination with a metal ion such as strontium ion, alginate in combination with a metal ion such as barium ion, and mixtures thereof, and more preferably scleroglucan, a mixture of microcrystalline cellulose (MCC) and carboxymethylcellulose (CMC), pectin in combination with strontium ion, HA-g-PNIPAm, and mixtures thereof,
- a semen extender as previously defined and preferably NutriXcell ultra, NutriXcell plus, OptiXcell, OptiXcell Alpha, and mixtures thereof,
- optionally an inhibitor of Slo3 potassium channel,
- optionally lipids such as liposomes, and
- optionally an additional compound as previously defined and preferably a mixture of sialic acid and lectin such as Wheat Germ Agglutinin (WGA).

### Anti-settling effect

The present inventors have surprisingly found that the composition as previously defined may be used to prevent sedimentation of sperm cells (anti-settling effect), and in particular by creating a weak network able to trap the sperm cells during storage.

When the composition is used to prevent sedimentation of sperm cells, the compound is preferably present at a concentration comprised between 0.001 % and 5 % by weight, preferably between 0.01 % and 4 % by weight, and more preferably between 0.1 % and 3 % by weight with respect to the total weight of the composition.

When the composition is used to prevent sedimentation of sperm cells, the compound is preferably a polysaccharide as previously defined and more preferably, a compound selected from gelatin, microcrystalline cellulose (MCC), methylcellulose, carboxymethylcellulose (CMC), hydroxymethylcellulose, ethylcellulose, hydroxyethylcellulose, hydroxypropylmethylcellulose (HPMC), pullulan, dextran, gum such as guar gum, xanthan gum, acacia gum, carrageenan gum, arabinogalactan gum, and mixtures thereof, scleroglucan, peptan, and mixtures thereof.

More preferably, when the composition is used to prevent sedimentation of sperm cells, the compound is selected from scleroglucan, microcrystalline cellulose (MCC), carboxymethylcellulose (CMC), and mixtures thereof such as a mixture of microcrystalline cellulose (MCC) and carboxymethylcellulose (CMC).

Preferably, the composition comprises:
- human and/or animal semen comprising sperm cells such as spermatozoa,
- a compound selected from collagen peptides, polymers and co-polymers including polysaccharides, and mixtures thereof as previously defined and preferably scleroglucan, microcrystalline cellulose (MCC), carboxymethylcellulose (CMC), a mixture of microcrystalline cellulose (MCC) and carboxymethylcellulose (CMC), and mixtures thereof,
- a semen extender as previously defined and preferably NutriXcell ultra, NutriXcell plus, OptiXcell, OptiXcell Alpha, and mixtures thereof,
- optionally an inhibitor of Slo3 potassium channel,
- optionally lipids such as liposomes, and
- optionally an additional compound as previously defined and preferably a mixture of sialic acid and lectin such as Wheat Germ Agglutinin (WGA).

### Gel effect

The present inventors have surprisingly found that the compositions of the present invention may form a gel to sequester the inhibitor of Slo3 potassium channel when present thereby increasing the contact time between the inhibitor of Slo3 potassium channel and sperm cells and potentiate its action on the sperm cells in particular during artificial insemination and its dilution in the uterus. Advantageously, the action time of the inhibitor is increased between the moment of Al and the arrival of the sperm cells near the oocyte, increasing the fertility time of an Al dose.

The present inventors have also surprisingly found that the compositions of the present invention may gradually release spermatozoa over time and in particular ensure a spread of sperm cells in the uterus over a longer period of time during artificial insemination (thereby increasing the insemination window by keeping sperm fertile longer).

Advantageously, the composition as previously defined may form a gel which is not toxic for the sperm cells but also for the uterus (and without physiological impact), which is capable of sequestering sperm cells and capable of releasing mobile sperm cells over time during an artificial insemination, in vitro fertilization (IVF) and/or intra-cytoplasmic sperm injection (ICSI) for example.

When the composition is used to form a gel, the compound is preferably present at a concentration comprised between 0.1 % and 10 % by weight, preferably between 0.5 % and 5 % by weight, and more preferably between 1 % and 3 % by weight with respect to the total weight of the composition.

When the composition is used to form a gel, the compound is preferably selected from methylcellulose, carboxymethylcellulose (CMC), hydroxymethylcellulose, ethylcellulose, hydroxyethylcellulose, hydroxypropylmethylcellulose (HPMC), alginate, pectin, pullulan, chitosan, hyaluronic acid (HA), dextran, gum such as guar gum, xanthan gum, acacia gum, carrageenan gum, arabinogalactan gum, and mixtures thereof, peptan, polymers and co-polymers such as ethylene glycol methacrylate polymer (PEGMA), triethylene glycol methacrylate polymer (PTEGMA), poly-oligo(ethylene glycol) methyl ether methacrylate polymer (POEGMA), N-isopropylacrylamide polymer (PNiPAM), Poly-N-vinylcaprolactam (PNVCL), poloxamers such as Pluronic^{®} F-127 (CAS 9003-11-6), poly(ethylene oxide-co-propylene oxide) polymer (PEPO), and mixtures thereof such as HA-g-PNIPAm and Alg-g-PNIPAm, more preferably HA-g-PNIPAm, Alg-g-PNIPAm, and mixtures thereof.

Preferably, the compound is selected from carboxymethylcellulose (CMC), alginate, pectin, chitosan, hyaluronic acid (HA), gum such as guar gum, xanthan gum, acacia gum, carrageenan gum, arabinogalactan gum, and mixtures thereof, in combination with a metal ion, preferably selected from beryllium ion, magnesium ion, calcium ion, strontium ion, barium ion, radium ion, and mixtures thereof, and mixtures thereof, and more preferably pectin in combination with a metal ion such as strontium ion or alginate in combination with a metal ion such as barium ion.

Preferably, the composition comprises:
- human and/or animal semen comprising sperm cells such as spermatozoa,
- a compound selected from collagen peptides, polymers and co-polymers including polysaccharides, and mixtures thereof as previously defined and preferably HA-g-PNIPAm, Alg-g-PNIPAm, pectin in combination with a metal ion such as strontium ion, alginate in combination with a metal ion such as barium ion, and mixtures thereof, and more preferably HA-g-PNIPAm, Alg-g-PNIPAm, pectin in combination with a strontium ion, and mixtures thereof,
- a semen extender as previously defined and preferably NutriXcell ultra, NutriXcell plus, OptiXcell, OptiXcell Alpha, and mixtures thereof,
- optionally an inhibitor of Slo3 potassium channel,
- optionally lipids such as liposomes, and
- optionally an additional compound as previously defined and preferably a mixture of sialic acid and lectin such as Wheat Germ Agglutinin (WGA).

The inhibitor of Slo3 potassium channel may be selected from barium, mibefradil, clofilium, quinidine, and mixtures thereof.

The inhibitor of Slo3 potassium channel may be present at a concentration ranging from 0.01 µM and 10 µM, preferably from 0.05 µM and 5 µM and more preferably from 0.1 µM and 2 µM.

Examples of lipids include, but are not limited to, liposomes such as liposomes from egg yolk phospholipids and liposomes from soy lecithin, and mixtures thereof.

The lipids such as liposomes may be present at a concentration ranging from 0.01 % to 10 % by weight, preferably from 0.05 % to 5 % by weight and more preferably from 0.1 % to 1 % by weight with respect to the total weight of the composition.

The animal semen may be selected from mammal semen such as bull, bovine, porcine, equine, goat, sheep, buffalo, camel, aquatic animals such as fish, crustaceans and mollusks, and insects, cat and/or dog semen, and avian semen such as chicken and/or turkey semen, preferably from avian, bovine and/or porcine semen and more preferably from bovine and/or porcine semen.

The composition as previously defined may further comprise a divalent or trivalent ion chelating agent, preferably a photocleavable divalent or trivalent ion chelating agent more preferably selected from o-nitrobenzyl based compounds such as o-nitrobenzyl-BAPTA and DMNP-EDTA, and mixtures thereof.

In particular, the present inventors have surprisingly found that the use of a divalent or trivalent ion chelating agent, in particular a photocleavable divalent or trivalent ion chelating agent, enables to sequester the divalent or trivalent ions and thus to prevent and/or limit the gelling process and/or increase in viscosity of the composition. Then, when the composition is exposed to an external stimuli (such as light illumination in a given wavelength range, pH, temperature, ionic force and combinations thereof) the chelated divalent or trivalent ions are released, the compound as previously defined cross-links, a gel is formed and/or the viscosity is increased which enables to traps the sperm cells thereby ensuring a controlled release of the sperm cells during insemination process, preferably in less than 72h and more preferably between 24 hours and 48 hours, but also increasing the contact time between the sperm cells and the inhibitor of Slo3 potassium channel when used.

Advantageously, the divalent or trivalent ion chelating agent as previously defined is not toxic for the semen and in particular the sperm cells but also for the uterus (and without physiological impact), has antioxidant and acrosome protective properties and may participate in the triggering of thickening properties and in the anti-sedimentation effect or may be used as adjuvant in gelling formulas for a controlled release, preferably in less than 72h and more preferably between 24 hours and 48 hours, of the sperm cells.

The chelating agent may be present at a concentration ranging from 0.005 % to 5 % by weight, preferably from 0.01 % to 3 % and more preferably from 0.05 % to 1 % by weight with respect to the total weight of the composition.

The ratio between the amount of semen and chelating agent may be comprised between 10 and 200, preferably between 20 and 180, and more preferably between 50 and 150.

Preferably, the composition contains the sperm cells in a dose, which is significantly lower than the dose used in conventional artificial insemination. More preferably, the dose is lower by a factor of at least 5, preferably by a factor of at least 10, more preferably by a factor of at least 20, more preferably by a factor of at least 30, more preferably by a factor of at least 50, and even more preferably by a factor of at least 100, compared to the dose used in conventional artificial insemination.

A dose used in conventional artificial insemination generally contains approximately between 0.2 billion and 3 billion of sperm cells, preferably between 2 million and 100 million of sperm cells.

In particular, when the sperm is selected from bovine sperm, the dose may contain approximately between 2 million and 100 million of bovine sperm cells.

In particular, when the sperm is selected from porcine sperm, the dose may contain approximately between 0.2 billion and 3 billion of porcine sperm cells.

The dose can comprise a volume of 0.25 mL to 100 mL.

For example, when the sperm is selected from bovine sperm, the dose may comprise a volume of 0.25 mL to 0.5 mL.

For example, when the sperm is selected from porcine sperm, the dose may comprise a volume between 10 mL and 100 mL.

Preferably, the composition comprises sperm cells at a dose comprised between 1 million and 300 million, more preferably between 2 million and 250 million.

In particular, when the sperm is selected from bovine sperm, the dose may comprise approximately 2 million of sperm cells per dose.

In particular, when the sperm is selected from porcine sperm, the dose may comprise approximately 250 million of sperm cells per dose.

The present invention also relates to a gel comprising the composition as previously defined.

When the composition is used to form a gel, the composition may have an elastic modulus comprised between 100 Pa and 10000 Pa.

When the composition is used to prevent sedimentation of sperm cells, the composition may have a viscosity comprised between 1 and 10000 mPa.s.

Preferably, the gel is a thermoresponsive gel (or temperature-responsive gel).

When the gel is a thermoresponsive gel (or temperature-responsive gel) or when the composition is used to form a thermoresponsive gel (or temperature-responsive gel), the compound as previously defined is preferably a polymer and/or a co-polymer (chain) as previously defined grafted to a polysaccharide as previously defined (backbone).

The composition may further comprise water.

The present invention also relates to a kit comprising the composition as previously defined.

The present invention also relates to a process for manufacturing the composition as previously defined or the gel as previously defined comprising the steps of:
- mixing the semen extender with the compound, optionally a metal ion, an inhibitor of Slo3 potassium channel and/or lipids such as liposomes, and optionally the additional compound and/or the divalent or trivalent ion chelating agent, to obtain a mixture,
- diluting said mixture in water to obtain a diluted mixture, and
- mixing the human and/or animal semen to said diluted mixture.

The present invention also relates to the use of the composition as previously defined or of the gel as previously defined to increase the contact time between the sperm cells and the inhibitor of Slo3 potassium channel.

The present invention also relates to the composition as previously defined or of the gel as previously defined for its use to increase the contact time between the sperm cells and the inhibitor of Slo3 potassium channel.

The present invention also relates to the use of the composition as previously defined or of the gel as previously defined for the manufacture of a medicament to increase the contact time between the sperm cells and the inhibitor of Slo3 potassium channel.

The present invention also relates to a method for increasing the contact time between the sperm cells and the inhibitor of Slo3 potassium channel comprising contacting sperm cells with the composition as previously defined or the gel as previously defined.

The present invention also relates to the use of the composition as previously defined or of the gel as previously defined to prevent sperm cells from sedimentation.

The present invention also relates to the composition as previously defined or to the gel as previously defined for its use to prevent sperm cells from sedimentation.

The present invention also relates to the use of the composition as previously defined or of the gel as previously defined for the manufacture of a medicament to prevent sperm cells from sedimentation.

The present invention also relates to a method for preventing sperm cells from sedimentation comprising contacting sperm cells with the composition as previously defined or the gel as previously defined.

The present invention also relates to the use of the composition as previously defined to inhibit devitrification of sperm during freezing, to prevent sperm cells from oxidation, to preserve the integrity of the acrosome of sperm cells and to improve the mitochondrial activity of sperm cells.

The present invention also relates to the use of the composition as previously defined or of the gel as previously defined to increase motility, viability and lifespan of sperm cells and in particular spermatozoa, preferably capacitated spermatozoa.

The present invention also relates to the composition as previously defined or to the gel as previously defined for its use to increase motility, viability and lifespan of sperm cells and in particular spermatozoa, preferably capacitated spermatozoa.

The present invention also relates to the use of the composition as previously defined or of the gel as previously defined for the manufacture of a medicament to increase motility, viability and lifespan of sperm cells and in particular spermatozoa, preferably capacitated spermatozoa.

The present invention also relates to a method for increasing motility, viability and lifespan of sperm cells and in particular spermatozoa, preferably capacitated spermatozoa, comprising contacting sperm cells with the composition as previously defined or the gel as previously defined.

The present invention also relates to the use of the composition as previously defined or of the gel as previously defined to increase fertility.

The present invention also relates to the composition as previously defined or to the gel as previously defined for its use to increase fertility.

The present invention also relates to the use of the composition as previously defined or of the gel as previously defined for the manufacture of a medicament to increase fertility.

The present invention also relates to a method for increasing fertility comprising contacting sperm cells with the composition as previously defined or the gel as previously defined.

The present invention also relates to the use of the composition as previously defined or of the gel as previously defined to treat infertility and in particular male infertility.

The present invention also relates to the composition as previously defined or to the gel as previously defined for its use to treat infertility and in particular male infertility.

The present invention also relates to the use of the composition as previously defined or of the gel as previously defined for the manufacture of a medicament to treat infertility and in particular male infertility.

The present invention also relates to a method for treating infertility, and in particular male infertility, comprising contacting sperm cells with the composition as previously defined or the gel as previously defined.

The present invention also relates to an artificial insemination instrument for use in artificial insemination (Al), in vitro fertilization (IVF) and/or intra-cytoplasmic sperm injection (ICSI) comprising the composition as previously defined or the gel as previously defined.

The present invention also relates to the use of the composition as previously defined or the gel as previously defined in artificial insemination (Al), in vitro fertilization (IVF) and/or intra-cytoplasmic sperm injection (ICSI) methods.

The present also relates to a method for artificially inseminating a human and/or an animal comprising the use of the composition as previously defined or the gel as previously defined.

By "method" and "use" as previously defined it may be understood either therapeutic or non-therapeutic method and either therapeutic or non-therapeutic use, either *in vitro* or *in vivo* method, either *in vitro* or *in vivo* use.

### BRIEF DESCRIPTION OF THE FIGURES

Figure 1 is a graph representing the sperm cell concentration, the sperm cell motility and the motile concentration of sperm cells in the tested samples at T=2H and T=4H.
Figure 2 is a graph representing the sperm cell motility in the tested samples at T=0, and T3H 37°C.
Figure 3 is a graph representing the sperm cell viability in the tested samples at T=0 and T3H 37°C.

### EXAMPLES

### Example 1 (anti-settling effect):

### Materials and methods:

Goal: assessment of sperm cells release at 37°C using a composition according to the present invention
- 4 samples of bull semen (from CIA - Coopérative Interdépartementale d'Elevage et d'Insemination Animale, L'Aigle) were pooled and diluted (around 200.10⁶ spz/mL (spermatozoa per mL)) in OptiXcell Alpha at 4°C,
- Thermoresponsive hydrogels made from HA-g-PNiPAm (at a concentration of 2%) were solubilized in OptiXcell Alpha at 4°C,

| | Backbon e | Backbon e molar mass (g/mol) | Grafts | Graft molar mass (g/mo l) | Ratio backbone/Gra fts | Tested concentrati on (% wt) |
|---|---|---|---|---|---|---|
| HA-g-PNiPA m | Hyaluron ic acid | 244000 | Poly(N-IsoPropylAcrylami de) | 30 000 | 27/73 | 2% |

- The samples were homogenized at 4°C,
- Mini-straws were filled and frozen,
- 6 Straws were emptied in a well plate and stored at 37°C,
- 1 mL of EHS (Embryo Holding Solution) was added in each well,
- The wells were incubated at 37°C,
- For the sperm release evaluation, the supernatant in the wells were pipetted and analysed,
- The analysis of sperm motility was performed on IVOS II ^{™} (using CASA - Computer Assisted Sperm Analysis - technology) at T=2H and T=4H

The control corresponds to OptiXcell Alpha alone.

### Results:

After emptying the wells: a gel was clearly observed.

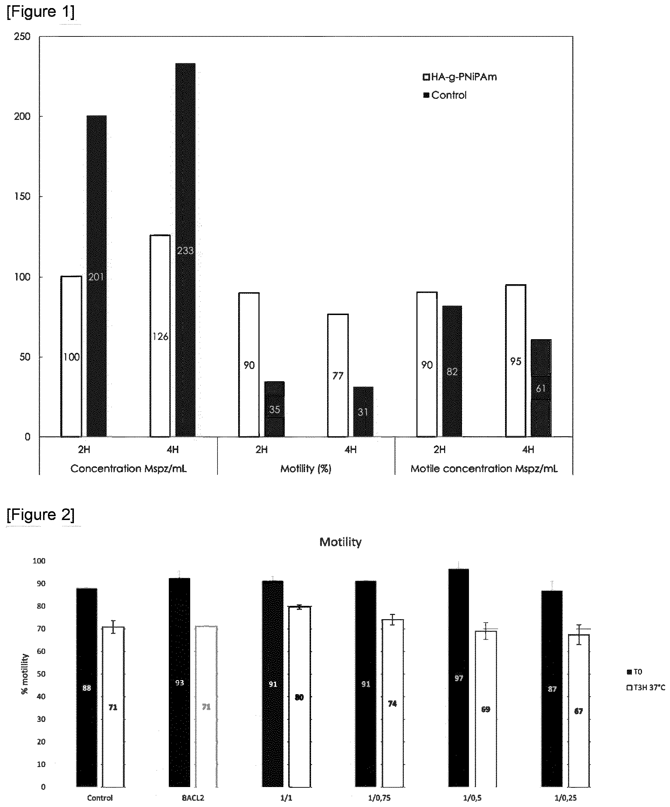

Results are shown on figure 1.

As shown on figure 1, HA-g-PNiPAm gels showed interesting motility results when pipetting at the up phase of the tube meaning that only motile sperm cells were analysed by the CASA. While for the control (OptiXcell Alpha alone), a motility of 35% was observed after 2 hours at 37°C. Concerning motile concentration the same trend was observed: HA-g-PNiPAm showed higher values, around 95 Mspz after 4 hours at 37°C while 61 Mspz/mL of motile concentration was analysed for the control.

It seemed that HA-g-PNiPAm released motile sperm cells over time.

### Conclusions:

A control release of sperm cells was successfully obtained over 4 hours.

### Example 2 (Photocleavable chelator):

The present inventors have found that DMNP-EDTA is a photocleavable chelator able to release divalent ions after illumination (external stimuli) to achieve a cross-linking of the alginate and to trap the spermatozoa.

### Materials and methods:

- DMNP-EDTA (PromoKiné),
- BaCl₂ (Sigma),
- Algaia alginate (with M/G ratio about 0.7-0.8),
- Water

| Compound | BaCl₂ | DMNP-EDTA | Algaia alginate (concentration 2.5%) |
|---|---|---|---|
| Molar mass (g/mol) | 208 | 762 | Approx. 200 000 |
| Amount of substance (pmol) | 6 | 6.6 | |
| Number of equivalent | 1 | 1.1 | |
| Concentration (mM) | 1.2 | 1.3 | |
| Mass (g) | | | 0.125 |
| Volume | 5mL (qsp water) | | |

- a 1.2 mM BaCl₂ solution in OptiXcell was prepared,
- 1 mL of this solution was resuspended in a 5 mg vial of DMNP-EDTA,
- 0.125 g of alginate were dispersed in the 5 mL of BaCl₂/DMNP-EDTA/Water solution,
- the gel was casted in a spectro cell, then illuminated with a UV lamp for 1 minute at 395 nm.

The control corresponds to non-illuminated solution.

### Results:

The gel was neither too viscous for casting, nor too liquid and had a good visual gelling.

After illumination, the Ba²⁺ ions have been released, the alginate cross-linking appeared.

By comparison, the control (non-illuminated solution) sunk, had a viscous liquid state while the flashed gel was more rigid, more elastic.

### Toxicity test - Material and method:

- a 2.4 mM BaCl₂ solution in OptiXcell was prepared,
- 1 mL of this solution was resuspended in a 5 mg vial of DMNP-EDTA,
- 1.5 mL of DMNP-EDTA were added for a final DMNP-EDTA concentration of 2.6 mM,
- The solution was kept away from light,
- The solution was contacted with semen in different chelator/semen ratios:
   1:1 => 500 µL chelators + 500 µL bull semen at 57 Mspz/mL
   1:0.75 => 500 µL chelators + 375 µL bull semen at 57 Mspz/mL
   1:0.5 => 500 µL chelators + 250 µL bull semen at 57 Mspz/mL
   1:0.25 => 500 µL chelators + 125 µL bull semen at 57 Mspz/mL
- A BaCl₂ solution only: 500 µL BaCl₂ at 2.4 mM + 500 µL bull semen at 57 Mspz/mL was prepared
- The following analyses have been performed on frozen bull semen:
   - Mobility analysis on IVOS II ^{™} at T=0 and T3H 37°C,
   - Acrosome integrity analysis on EasyCyte at T=0 and T3H 37°C,
   - Viability analysis on EasyCyte at T=0 and T3H 37°C
   - Mitochondrial activity analysis on EasyCyte at T=0 and T3H 37°C

The control corresponds to OptiXcell alone.

### Results - Motility and viability:

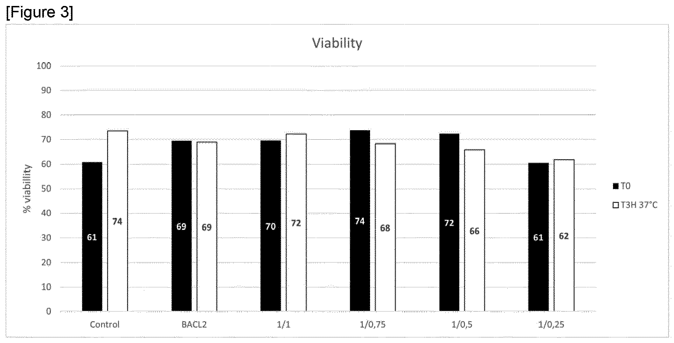

Results are shown on figures 2 and 3.

As shown on figure 2, the sperm motility was not affected by the presence of the chelator. At T=3H the 1/1 (chelator/semen) condition was better than the control (+9 pts) (OptiXcell alone).

As shown on figure 3, the presence of the chelator showed a positive effect on sperm viability at T=0. At T=0, the 1/0.75 condition proved to be the best in viability (+13 pts vs. Control). At T=3H, the range effect appeared, viability was affected as the chelator/sperm ratio decreased. However, the BaCl₂ condition remained unchanged.

### Conclusions:

No major impact on mobility and viability in the presence of chelator at T0 and T3H 37°C was observed.

## Claims

1. Composition comprising:
- human and/or animal semen comprising sperm cells such as spermatozoa,
- a compound selected from collagen peptides, polymers and co-polymers including polysaccharides, and mixtures thereof,
- a semen extender,
- optionally an inhibitor of Slo3 potassium channel, and
- optionally lipids such as liposomes.

2. Composition according to claim 1, wherein polymers and co-polymers are selected from anionic polymers and co-polymers including anionic polysaccharides, cationic polymers and co-polymers including cationic polysaccharides, non-ionic polymers and co-polymers including non-ionic polysaccharides, polymers and co-polymers covalently grafted such as polymers and co-polymers grafted with an amine function, thermoresponsive or temperature-responsive polymers and co-polymers such as LCST polymers and copolymers and UCST polymers and copolymers including polymers and co-polymers grafted from polysaccharides, grafted on polysaccharides or grafted through polysaccharides, and mixtures thereof.

3. Composition according to claim 1 or 2, wherein polymers and co-polymers are selected from polysaccharides, gelatin, ethylene glycol methacrylate polymer (PEGMA), triethylene glycol methacrylate polymer (PTEGMA), poly-oligo(ethylene glycol) methyl ether methacrylate polymer (POEGMA), N-isopropylacrylamide polymer (PNiPAM), Poly-N-vinylcaprolactam polymer (PNVCL), poloxamers such as Pluronic^{®} F-127 (CAS 9003-11-6), polyethylene oxide-co-propylene oxide) polymer (PEPO), polymers and co-polymers covalently grafted such as polymers and co-polymers grafted with an amine function including amino-terminated poloxamers, amino-terminated Poly(N-isopropylacrylamide) (PNiPAM) such as amino-terminated Poly(N-isopropylacrylamide) (PNiPAM) using cysteamine, amino terminated polyethylene oxide-co-propylene oxide) (PEPO), amino-terminated Poly(N-vinylcaprolactam) (PNVCL), amino-terminated ethylene glycol methacrylate polymer (PEGMA), amino terminated poly-oligo(ethylene glycol) methyl ether methacrylate polymer (POEGMA), amino-terminated polyoxazolines, and mixtures thereof.

4. Composition according to anyone of claims 1 to 3, wherein polysaccharides are selected from gum such as guar gum, xanthan gum, acacia gum, carrageenan gum, arabinogalactan gum, gellan gum, and mixtures thereof, cellulose such as microcrystalline cellulose (MCC) and cellulose derivatives such as methylcellulose, carboxymethylcellulose (CMC), hydroxymethylcellulose, ethylcellulose, hydroxyethylcellulose, hydroxypropylmethylcellulose (HPMC), and mixtures thereof, alginate such as sodium alginate, pectin, pullulan, chitosan, hyaluronic acid (HA), dextran, scleroglucan, and mixtures thereof.

5. Composition according to anyone of claims 1 to 4, wherein the composition further comprises a metal ion, preferably a divalent and/or trivalent metal ion, more preferably selected from beryllium ion, magnesium ion, calcium ion, strontium ion, barium ion, radium ion, and mixtures thereof.

6. Composition according to any one of claims 1 to 5, wherein the compound is present at a concentration comprised between 0.001 % and 10 % by weight, preferably between 0.01 % and 10 % by weight, and more preferably between 0.1 % and 5 % by weight with respect to the total weight of the composition.

7. Composition according to any one of claims 1 to 5, wherein the compound is present at a concentration comprised between 0.001 % and 5 % by weight, preferably between 0.01 % and 4 % by weight, and more preferably between 0.1 % and 3 % by weight with respect to the total weight of the composition.

8. Composition according to claim 7, wherein the compound is selected from gelatin, microcrystalline cellulose (MCC), methylcellulose, carboxymethylcellulose (CMC), hydroxymethylcellulose, ethylcellulose, hydroxyethylcellulose, hydroxypropylmethylcellulose (HPMC), pullulan, dextran, gum such as guar gum, xanthan gum, acacia gum, carrageenan gum, arabinogalactan gum, and mixtures thereof, scleroglucan, peptan, and mixtures thereof.

9. Composition according to any one of claims 1 to 5, wherein the compound is present at a concentration comprised between 0.1 % and 10 % by weight, preferably between 0.5 % and 5 % by weight, and more preferably between 1 % and 3 % by weight with respect to the total weight of the composition.

10. Composition according to any one of claims 9, wherein the compound is selected from methylcellulose, carboxymethylcellulose (CMC), hydroxymethylcellulose, ethylcellulose, hydroxyethylcellulose, hydroxypropylmethylcellulose (HPMC), alginate, pectin, pullulan, chitosan, hyaluronic acid (HA), dextran, gum such as guar gum, xanthan gum, acacia gum, carrageenan gum, arabinogalactan gum, and mixtures thereof, peptan, polymers and co-polymers such as ethylene glycol methacrylate polymer (PEGMA), triethylene glycol methacrylate polymer (PTEGMA), poly-oligo(ethylene glycol) methyl ether methacrylate polymer (POEGMA), N-isopropylacrylamide polymer (PNiPAM), Poly-N-vinylcaprolactam (PNVCL), poloxamers such as Pluronic^{®} F-127 (CAS 9003-11-6), polyethylene oxide-co-propylene oxide) polymer (PEPO), and mixtures thereof.

11. Composition according to claim 9, wherein the compound is selected from carboxymethylcellulose (CMC), alginate, pectin, chitosan, hyaluronic acid (HA), gum such as guar gum, xanthan gum, acacia gum, carrageenan gum, arabinogalactan gum, and mixtures thereof, in combination with a metal ion, preferably selected from beryllium ion, magnesium ion, calcium ion, strontium ion, barium ion, radium ion, and mixtures thereof.

12. Composition according to any one of claims 1 to 11, wherein the composition further comprises a divalent or trivalent ion chelating agent, preferably a photocleavable divalent or trivalent ion chelating agent more preferably selected from o-nitrobenzyl based compounds such as o-nitrobenzyl-BAPTA and DMNP-EDTA, and mixtures thereof.

13. Composition according to claim 12, wherein the chelating agent is present at a concentration ranging from 0.005 % to 5 % by weight, preferably from 0.01 % to 3 % by weight and more preferably from 0.05 % to 1 % by weight with respect to the total weight of the composition.

14. Process for manufacturing the composition as defined in any one of claims 1 to 13 comprising the steps of:
- mixing the semen extender with the compound, optionally a metal ion, an inhibitor of Slo3 potassium channel and/or lipids such as liposomes, and optionally the additional compound and/or the divalent or trivalent ion chelating agent, to obtain a mixture,
- diluting said mixture in water to obtain a diluted mixture, and
- mixing the human and/or animal semen to said diluted mixture.

15. Use of the composition as defined in anyone of claims 1 to 13 to increase the contact time between the sperm cells and the inhibitor of Slo3 potassium channel.

16. Use of the composition as defined in anyone of claims 1 to 13 to prevent sperm cells from sedimentation.

17. Composition as defined in anyone of claims 1 to 13 for its use to increase motility, viability and lifespan of sperm cells and in particular spermatozoa, preferably capacitated spermatozoa.

18. Composition as defined in anyone of claims 1 to 13 for its use to increase fertility.
